# EUROPEAN PATENT APPLICATION

(11) **EP 1 882 538 A2**
(43) Date of publication of application: **30.01.2008**
(21) Application number: 07252974.6
(22) Date of filing: 27.07.2007
(51) Int. Cl.: B23D 49/10, A61B 17/00, A61B 17/16, A61B 17/14

(54) **Orthopaedic bone saw and bone drill adapters**

(30) Priority: 28.07.2006 US 460733
(71) Applicant: DePuy Products, Inc., Warsaw, IN 46581 (US)
(72) Inventor: Gundlapalli, RamRao V, IN 46539 (US); Smith, Matthew D., IN 46910 (US); Mongeau, Jean-Michel, NY 12186 (US); Sedgwick, Joseph A., OH 45248 (US); Martin, Troy D., IN 46562 (US); Suh, Phil Y., Warsaw, IN 46582 (US); Long, Jack F., Warsaw, IN 46582 (US)
(74) Representative: Belcher, Simon James

(57) **Abstract**

Adapters (10,110,210) for oscillating bone saws and bone drills can convert the output motion of the bone saws (12) and the bone drills (250), for example converting the oscillating motion of bone saws to a modified oscillating motion or to an orbital motion, and converting rotational motion of bone drills to an orbital motion.

## Description

The present disclosure relates generally to orthopaedic surgical tools used on long bones and during joint replacement surgeries such as oscillating bone saws and bone drills. Specifically, the present disclosure relates to adapters for use with bone saws and bone drills to change the output motion of each.

Orthopaedic surgical procedures often involve cutting, trimming, drilling, and/or shaving of bone structures such as long bones and/or joint-type bones, for example. Long bones are hard, dense bones that generally provide strength, structure, and mobility. Long bones include the femur, tibia, and fibula of the leg, the humerus, radius, and ulna of the arm, and the phalange of the finger and toe, for example. Oscillating bone saws are used to prepare such bones to receive and properly align an orthopaedic implant during various orthopaedic surgical procedures such as total or partial joint replacement surgeries, for example, where some or all of an arthritic or damaged joint is replaced by an artificial joint. Examples of bone saws are disclosed in US-3905105, US-3977289, US-6949110, US-6302406 and US-A-2006/0009796.

Such bone structures are surrounded by soft tissue such as muscle, cartilage, tendons, and ligaments, for example. These soft tissue structures may be difficult to isolate during orthopaedic procedures which involve the cutting of bone structures. For example, surgeons use instruments such as retractors to move the soft tissue away from the operating site to provide both proper visualization of the bone and also to prevent any inadvertent damage to the soft tissue. However, the soft tissue remains attached to the bony structures and may only be retracted a finite amount. As such, the surrounding soft tissue may be unintentionally damaged by the oscillating bone saw during the orthopaedic surgical procedure. The issue of soft tissue damage is discussed in Da Silva, M.A., et al., "Popliteal Vascular Injury during Total Knee Arthroplasty," Journal of Surgical Research, Volume 109:2, February 2003, pp. 170-174; Krackow, K.A., "MCL Repair and Reconstruction," The Knee Society Specialty Day Meeting, Feb. 8, 2003; and Leopold, S.S., et al., "Primary Repair of Intraoperative Disruption of the Medial Collateral Ligament During Total Knee Arthroplasty," The Journal of Bone & Joint Surgery, Volume 83-A:1, January 2001.

In one aspect, the invention provides an oscillating bone saw assembly includes an oscillating bone saw having a drive mechanism and a bone saw blade coupled to the drive mechanism. The arc length of the oscillating motion of the distal end of the bone saw blade is at least about 4.4 mm (0.175 inch) or not more than 5.6 mm (0.220 inch), or preferably from about 4.4 mm to about 5.6 mm. Further, the length between a pivot point of the bone saw blade and the distal end of the bone saw blade may be approximately 89 mm (3.5 inch) or less. The oscillating motion of the bone saw blade may define an angle of approximately 3.6° or less.

In another aspect, the invention provides an orbital bone saw assembly for producing an orbital output motion includes a drive mechanism and a bone saw blade coupled to the drive mechanism. Preferably, the orbital output motion of the blade defines a major chord having a length of at least about 4.4 mm (0.175 inch) or not more than 5.6 mm (0.220 inch), or preferably from about 4.4 mm to about 5.6 mm. Further, the longitudinal axis of the blade may be perpendicular to the major chord defined by the orbital output motion of the blade. Preferably, the orbital bone saw assembly may include an oscillating bone saw or a rotating bone drill. As such, one of the bone saw and bone drill includes the drive mechanism and the blade clamp of the orbital bone saw assembly.

The instruments of the invention can be used in method for operating an oscillating bone saw assembly includes coupling a bone saw blade to a drive mechanism of an oscillating bone saw and oscillating the blade such that the arc length of the oscillating motion of the distal end of the blade is at least about 4.4 mm (0.175 inch) or not more than 5.6 mm (0.220 inch), or preferably from about 4.4 mm to about 5.6 mm. Oscillating the blade may include modifying the output motion of the oscillating bone saw by coupling an adapter to the drive mechanism of the oscillating saw and coupling the blade to the adapter. Further, the output motion of the drive mechanism may define an angle of approximately 8° and the output motion of the blade may define an angle of approximately 3.6° or less. A hub connector may be coupled to the drive mechanism about a first axis, the blade may be coupled to the hub connector, and the blade may be oscillated about a second axis that is spaced-apart from the first axis.

The instruments of the invention can be used in a method for operating an orbital bone saw assembly includes coupling a bone saw blade to a drive mechanism of either an oscillating bone saw or a bone drill and moving a distal end of the blade to define an orbital path having a major chord of at least about 4.4 mm (0.175 inch) or not more than 5.6 mm (0.220 inch), or preferably from about 4.4 mm to about 5.6 mm. An adapter may be coupled to the drive mechanism of either the oscillating bone saw or the bone drill. Further, the blade may be coupled to the adapter. A pivot point of the blade located at a proximal end of the blade may be spaced-apart from an axis of rotation about which the blade rotates.

In another aspect, the invention provides an adapter which is configured to be coupled to a blade clamp of an oscillating bone saw for modifying oscillating output motion of the oscillating bone saw includes a housing configured to be coupled the blade clamp, a hub connector configured to be coupled to a hub of the blade clamp, and a bone saw blade pivotably coupled to the housing to define a pivot point. Preferably, a proximal end of the blade is coupled to the hub connector. The hub connector may include gear teeth and the proximal end of the blade may also include gear teeth interlocked with the gear teeth of the hub connector. As such, the gear ratio between the blade and the hub connector may be approximately 2:1. Further, the length of the blade from the pivot point to the distal end of the blade may be approximately 89 mm (3.5 inch) or less.

In another aspect, the invention provides an adapter which is configured to be coupled to an oscillating bone saw for converting oscillating output motion of the oscillating bone saw to an orbital motion includes a housing configured to be coupled to the blade clamp of the oscillating bone saw, a driven mechanism coupled to the housing and configured to be coupled to a drive mechanism of the blade clamp of the oscillating bone saw, and a blade coupled to the driven mechanism. The driven mechanism may include a hub connector configured to be coupled to a drive mechanism of the oscillating bone saw, a linkage mechanism coupled at a first end to the hub connector, and a gear assembly coupled to a second end of the linkage mechanism. The driven mechanism may further include an output shaft coupled to the gear assembly and received through an aperture of the blade.

Preferably, the gear assembly includes a first gear coupled to the linkage mechanism and a second gear coupled to the first gear. The output shaft may be coupled to the second gear and may be spaced-apart from an axis about which the second gear rotates. Further, the blade may include a slot and the housing may include a guide pin received within the slot of the blade. The longitudinal axis of the slot may be parallel to a longitudinal axis of the blade. Preferably, the distance between the output shaft and the axis of rotation may be less than the distance between the axis of rotation and the guide pin. Further, the distance between the axis of rotation and the guide pin may be less than a distance between the aperture of the blade and a distal end of the blade.

Preferably, the adapter is configured to produce an output motion of the distal end of the blade which defines a major axis having a length of at least about 4.4 mm (0.175 inch) or not more than 5.6 mm (0.220 inch), or preferably from about 4.4 mm to about 5.6 mm.

In another aspect, the invention provides an adapter which is configured to be coupled to a chuck of a bone drill for converting rotational output motion of the bone drill to an orbital motion includes a first beveled gear configured to be coupled to an output drive shaft of the chuck of the bone drill to define a first axis of rotation. A second beveled gear of the adapter is coupled to the first beveled gear and positioned to define a second axis of rotation perpendicular to the first axis of rotation. A blade of the adapter includes a proximal end coupled to the second beveled gear to define a pivot point of blade. The pivot point of the blade may be spaced-apart from the second axis of rotation of the second bevelled gear.

Preferably, the adapter includes a housing such that the first and second beveled gears are positioned within the housing. The housing may include a guide pin positioned within a slot of the blade. The longitudinal axis of the slot may be parallel to a longitudinal axis of the blade. Preferably, a first distance between the second axis of rotation and the guide pin may be greater than a second distance between the second axis of rotation and the pivot point of the blade. A length of the slot may be at least two times the second distance plus a diameter of the guide pin. The first distance between the second axis of rotation and the guide pin may be less than a third distance between the pivot point of the blade and a distal end of the blade.

The adapter may be configured to produce an output motion of the distal end of the blade which defines a major chord having a length of at least about 4.4 mm (0.175 inch) or not more than 5.6 mm (0.220 inch), or preferably from about 4.4 mm to about 5.6 mm. The major chord of the output motion may be perpendicular to the longitudinal axis of the blade.

In another aspect, the invention provides an adapter which is configured to be coupled to a blade clamp of an oscillating bone saw in order to modify the oscillating output motion of the oscillating bone saw includes a bone saw blade having an opening configured to receive a detent of the blade clamp therein. The opening may be sized such that a clearance of at least 0.2 mm (0.008 inch) exists between an outer surface of the detent and a corresponding surface of the opening. The opening may be generally circular to a first diameter and the detent may also be generally circular to defines a second diameter. Preferably, the first diameter may be at least 0.2 mm (0.008 inch) greater than the second diameter.

An adjuster plate of the adapter may be coupled to the bone saw blade and may be movable relative to the bone saw blade between a first position and a second position. The adjuster plate may include a generally tear-drop shaped slot having a longitudinal axis parallel to the longitudinal axis of the bone saw blade. Preferably, a proximal end of the slot defines a first width greater than a second width of a distal end of the slot. The first width of the proximal end of the slot may be greater than or equal to the diameter of the opening of the bone saw blade while the second width of the distal end of the slot may be less than the diameter of the opening of the bone saw blade.

Preferably, the opening of the bone saw blade is aligned with a distal end of the slot of the adjuster plate when the adjuster plate is positioned in the first position and is aligned with a proximal end of the slot of the adjuster plate when the adjuster plate is positioned in the second position. The bone saw blade may include a plurality of openings and the adjuster plate may include a plurality of slots.

The adapter may further include a knob coupled to the adjuster plate and movable relative to the bone saw blade to move the adjuster plate between the first and second positions. The knob may include a head and a threaded stem received through (i) an elongated slot of the bone saw blade and (ii) a threaded aperture of the adjuster plate. Preferably, the knob can be moved in a direction parallel to the longitudinal axis of the bone saw blade in order to move the adjuster plate between the first and second positions. Alternatively, the knob can be rotated relative to the bone saw blade about a pivot point in order to move the adjuster plate between the first and second positions.

The instruments of the invention can be used in a method of operating an oscillating bone saw assembly includes coupling a bone saw blade to a drive mechanism of a blade clamp of an oscillating bone saw, oscillating the drive mechanism about a pivot point through a first angle of motion, and oscillating the bone saw blade about the pivot point through a second angle of motion. The second angle of motion is less than the first angle of motion. A plurality of detents of the oscillating bone saw may be received through a plurality of openings of the bone saw blade in order to couple the bone saw blade to the drive mechanism of the bone saw.

The instruments of the invention can be used in a method of operating an oscillating bone saw assembly includes coupling a bone saw blade to a chuck of a drive mechanism of an oscillating bone saw such that a detent of the oscillating bone saw is received through an opening of the bone saw blade, moving the detent about a pivot point relative to the bone saw blade, and moving the detent and the bone saw blade about the pivot point.

Adapters provided by the present invention can modify or convert the output motion of the bone saw and/or bone drill to which each is attached in order to modify the output motion of a bone saw blade. For example, restricting the arc length of the oscillating motion of the distal end of a conventional bone saw blade may aide in preventing the blade from damaging soft tissues of the patient in the general area where the surgeon is cutting the patient's bone. Soft tissue may include veins, arteries, ligaments, tendons, cartilage, and muscle tissue, for example, as well as other soft tissues which may be damaged during orthopaedic procedures. Similarly, restricting the major chord of the orbital motion of the distal end of a convention bone saw blade may also aide in preventing the blade from damaging such soft tissues.

Conventionally, it had been thought that the sharpness of the blade and/or the rate at which the blade was moving were the main, if not the only, factors which contributed to the tearing or shredding of soft tissue during orthopaedic surgical procedures. Contrary to this school of thought, however, it has been found that the actual motion of the blade itself is the overriding factor relating to or causing the patient's surrounding soft tissues to be torn or shredded. In other words, limiting the motion of the blade to an amount which does not stretch the soft tissue beyond the elongation failure of the soft tissue may significantly reduce the amount of soft tissue which is torn. The elongation failure of a soft tissue may generally refer to the amount of stretching or deformation of the soft tissue which causes the soft tissue to tear. As such, an amount of deformation less than the elongation failure of a particular soft tissue will generally prevent the tissue from tearing. Of course, despite the factors discussed above which may contribute the damage of surrounding soft tissues, the surgeon operating the bone saw is primarily responsible for preventing such damage from occurring. However, the concepts disclosed herein may assist the surgeon in doing so.

It has been found that restricting the deformation or stretching of the soft tissue to 5.6 mm (0.220 inch) or less maintains the integrity of the soft tissue without approaching the elongation failure of the soft tissue. In other words, restricting the deformation of the soft tissue to 5.6 mm (0.220 inch) or less significantly reduces the amount of soft tissues which become torn. Of course, it is understood that various soft tissue structures within the body each have varying elongation failure rates. However, it has been found that by restricting the deformation of the soft tissue to 5.6 mm (0.220 inch) or less significantly reduces the amount of failure for all soft tissues surrounding the patient's bone.

Of course, the motion of the blade must also be sufficient to actually cut through the patient's bone. In other words, a minimum motion of the blade is required in order to effectively cut through the patient's bone. As such, it has been found through experimentation that a range of motion of the blade of at least 4.4 mm (0.175 inch) is able to satisfactorily cut through the patient's bone. Therefore, one exemplary range of motion of the blade of an oscillating bone saw assembly which effectively cuts through bone while reducing the potential of damaging surrounding soft tissue into which it may come into contact defines an arc length of 4.4 mm to 5.6 mm (0.175 to 0.220 inch) through which a distal end of the blade travels. Similarly, the orbital motion of a bone saw blade of an orbital bone saw assembly may also be restricted to reduce damage to surrounding soft tissue such that a major chord defined by the orbital motion of the distal end of the blade is 4.4 mm to 5.6 mm as well. The range is further limited to 4.4 mm to 5.6 mm in certain exemplary embodiments. However, adapters might also be useful which are configured such that an arc length of the oscillating motion of the blade or a major chord of the orbital motion of the blade is greater than 5.6 mm (0.220 inch) or smaller than 0.175 inch.

The adapters disclosed herein are retrofit mechanisms provided for use with an originally manufactured tool such as an OEM oscillating bone saw or an OEM rotating bone drill in order to modify the output motion of such saw or drill. In particular, the adapters described herein are configured to be coupled to a tool-retention mechanism, such as a blade clamp of an oscillating bone saw or a chuck (whether adjustable or non-adjustable, such as a socket) of a rotating bone drill, for example. As such, an adapter which modifies the output motion of an OEM tool (such as a bone saw or bone drill, for example) is distinct from any particular component of such OEM tool because while such tools may include internal drive mechanisms which modify the particular motion between one component and other, such components are not configured to be externally coupled to a tool-retention mechanism.

Embodiments of the invention will now be described by way of example with reference to the accompanying drawings, in which:
FIG. 1 is a perspective view of an example of a prior art oscillating bone saw and saw blade configured to be coupled to the oscillating bone saw;
FIG. 2 is a perspective view of a bone saw adapter coupled to the oscillating bone saw of FIG. 1 in order to modify the oscillating output motion of the bone saw;
FIG. 3 is an exploded, perspective view of the adapter of FIG. 2;
FIG. 4 is a top view of a portion of the adapter of FIGS. 2 and 3;
FIG. 5 is an exploded, perspective view of another bone saw adapter configured to be coupled to the oscillating bone saw of FIG. 1 in order to convert the oscillating output motion of the bone saw to orbital motion;
FIG. 6 is a bottom, exploded, perspective view of the adapter of FIG. 5;
FIG. 7 is a bottom view of the adapter of FIGS. 5 and 6;
FIG. 8 is a top view of the adapter of FIGS. 5 to 7;
FIG. 9 is a perspective view of an example of a prior art bone drill with an attachment configured to be coupled to the bone drill;
FIG. 10 is a perspective view of a bone drill adapter configured to be coupled to the bone drill shown in FIG. 9 in order to convert the rotational output motion of the bone drill to an orbital motion;
FIG. 11 is an exploded, perspective view of the bone drill adapter of FIG. 10;
FIG. 12 is a part-sectional, part-side view of the adapter of FIGS. 9 to 11;
FIGS. 13 and 14 are top views of the adapter of FIGS. 9 to 12 showing the orbital motion of a blade of the adapter;
FIG. 15 is a diagrammatic view showing oscillating motion of a saw blade;
FIG. 16 is a diagrammatic view showing orbital motion of a saw blade;
FIG. 17 is a top, perspective view of an oscillating bone saw adapter including a bone saw blade and a dual-mode adjuster plate;
FIG. 18 is a bottom, perspective view of a portion of the adapter of FIG. 17 showing the adjuster plate in a first position relative to the blade;
FIG. 19 is a bottom, perspective view of a portion of the adapter similar to FIG. 18 showing the adjuster plate in a second position relative to the blade;
FIG. 20 is a sectional view of a portion of the adapter of FIGS. 17 to 19;
FIG. 21 is a top, perspective view of another oscillating bone saw adapter including a bone saw blade and a dual-mode adjuster plate;
FIG. 22 is a bottom, perspective view of the adapter of FIG. 21 showing the adjuster plate in a first position relative to the blade;
FIG. 23 is a top view of a bone saw blade having cut-out portions along the length of the blade; and
FIG. 24 is a top view of another bone saw blade having cut-out portions similar to those shown in FIG. 23 along the length of the blade.

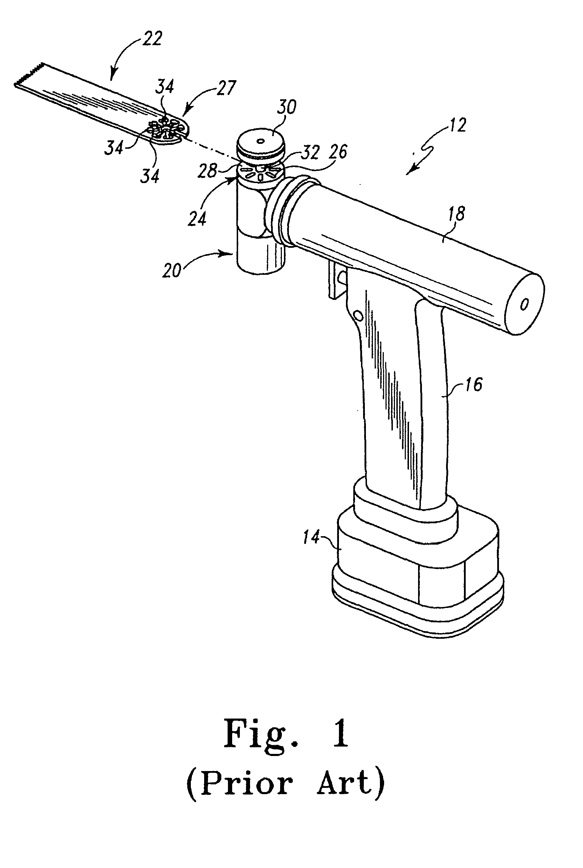

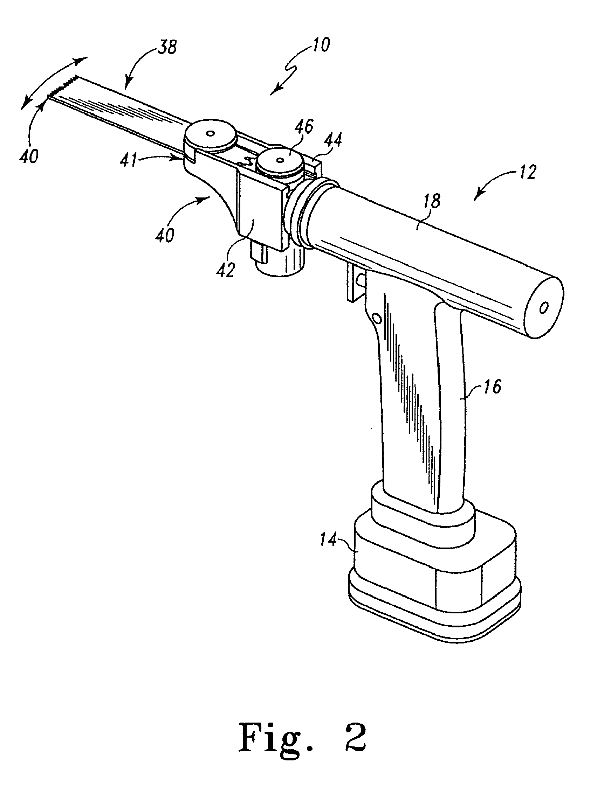

Referring to the drawings, FIGS. 1 and 2 show a bone saw adapter 10 which can be coupled to a conventional oscillating bone saw 12 in order to modify the oscillating output motion of the bone saw 12. The bone saw adapter 10 and the bone saw 12 cooperate to provide a bone saw assembly for cutting a patient's bone as is discussed in greater detail below. Preferably, the bone saw 12 includes a battery pack 14 coupled to a grip portion 16 and a head portion 18 coupled to the grip portion 16. While the battery pack 14 is shown, it is within the scope of this disclosure for the bone saw 12 to be powered via pneumatic source (not shown) as well. A blade clamp 20 of the saw 12 is coupled to the head portion 18 and is provided to receive a saw bone saw blade 22 (as shown in FIG. 1) in order to couple the blade 22 to the bone saw 12. The blade clamp 20 may be considered a tool (or blade) retention mechanism of the saw 12.

As is used herein, the term "bone saw blade" is defined as a saw blade used to cut long bones such as the femur, tibia, fibula, humerus, radius, ulna, and the phalange of the finger and toe, for example. Further, the term "bone saw blade" refers to a saw blade used during total or partial orthopaedic joint replacement surgeries such as, for example, hip replacement surgeries, knee replacement surgeries, wrist replacement surgeries, shoulder replacement surgeries, etc. As such, the bone saw blades discussed herein are contrasted from other saw blades used in dentistry or ear, nose, and throat (ENT) type surgeries.

The blade clamp 20 includes a hub 24 having an array of detents 26 protruding from a top surface 28 of the hub 24. A cover 30 is coupled to the hub 24 by a central post 32, as shown in FIG. 1. The blade clamp 20 is movable between an opened position such that the cover 30 is spaced-apart from the top surface 28 of the hub 24 (as shown in FIG. 1) and a closed position such that the cover 30 is adjacent to the top surface 28 of the hub 24 (as shown in FIG. 2). Looking now to FIG. 1, a proximal end 27 of the blade 22 includes an array of slots 34 formed to receive the array of detents 26 of the blade clamp 20 when the blade clamp is in the opened position. Once the blade 22 is received within the blade clamp 20 such that the detents 26 of the blade clamp 20 are received through the slots 28 of the blade 22, the blade clamp 20 may be moved to the closed position (as shown in FIG. 2) to secure the blade 22 to the saw 12.

Looking now to FIG. 2, the adapter 10 modifies the oscillating output motion of the bone saw 12. In particular, the adapter 10 operates to reduce an angle through which a bone saw blade 38 oscillates as compared to an angle through which the blade 22 oscillates when coupled directly to the saw 12. As such, an arc length through which a distal end 64 of the blade 38 travels is reduced as well. Typically, oscillating bone saws, such as the bone saw 12, may be used during various orthopaedic procedures such as minimally invasive surgery (MIS), computer-assisted surgery (CAS), arthroscopy, as well as open orthopaedic procedures. As is discussed in greater detail below, the arc length of the blade 38 of the adapter 10 is between 4.4 and 5.6 mm.

The adapter 10 may be configured to be coupled to any suitable oscillating bone saw such as, for example, the 7600 oscillating saw sold by MicroAire Surgical Instruments LLC of Charlottesville, VA and the pneumatic oscillating saws sold by ConMed Linvatec of Largo, Florida under the trade mark Hall PowerPro . The bone saw adapter 10 can be configured to be coupled to any conventional orthopaedic oscillating saw typically used during any orthopaedic surgical procedure.

Looking now to FIGS. 2 to 4, the adapter 10 includes a generally "U-shaped" housing 40 having a head portion 41 as well as first and second arms 42, 44 coupled to the head portion 41 and configured to be coupled to the blade clamp 20 of the saw 12, as shown in FIG. 2, for example. Preferably, the arms 42, 44 of the housing 40 may include threaded apertures (not shown) for receiving screws (not shown) therein to couple the adapter 10 to the blade clamp 20. A cap 46 is coupled to the head portion 41 to maintain the blade 38 between a blade-support surface 47 of the head portion 41 and the cap 46.

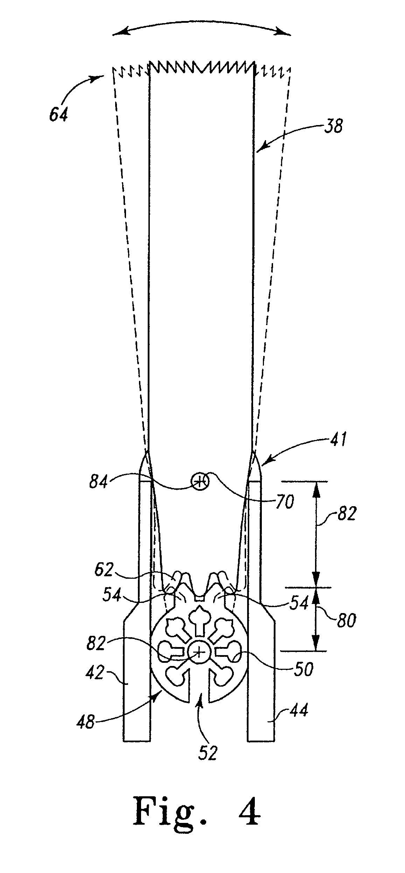

The adapter 10 further includes a hub connector 48 including an array of slots 50 similar to the array of slots 34 formed in the proximal end 27 of the blade 22 shown in FIG. 1. As such, the hub connector 48 is able to receive the array of detents 24 of the blade clamp 20 of the saw 12 in order to couple the connector 48 to the saw 12. Specifically, a center slot 52 of the hub connector 48 receives the vertical post 32 of the blade clamp 20 to operate as the pivot point 82 (see FIG. 4) about which the hub connector 48 oscillates. The hub connector 48 is generally circular in shape and includes a pair of gear teeth 54. Preferably, each arm 42, 44 of the housing 40 includes a channel 56 formed therein. As such, the hub connector 48 is received within the channels 56 to couple the hub connector 48 to the housing 40. As is discussed in greater detail below, the hub-connector 48 is able to oscillate about pivot point 82 relative to the housing 40.

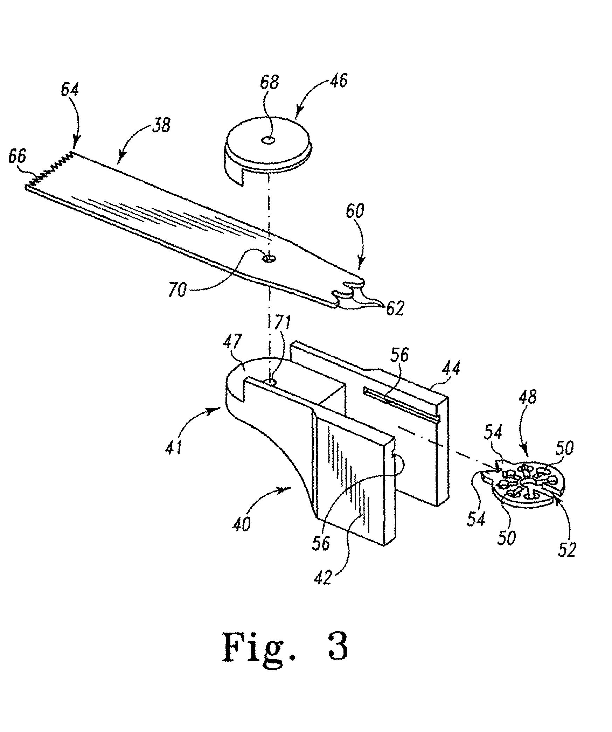

FIG. 3 shows the blade 38 with a proximal end 60 having gear teeth 62 and a distal end 64 having saw teeth 66. The blade 38 is positioned between the head portion 41 and the cap 46 of the housing 40 such that a portion of the blade 38 is supported on the surface 47 of the head portion 41 of the housing 40. A pin 68 is received through an aperture 70 of the blade 38 and an aperture 71 formed in the blade-support surface 47 to secure the blade 38 against pivotal movement relative to the housing 40. As such, the blade 38 can be pivoted relative to the housing 40 about the pin 68 to produce back and forth oscillating motion. The gear teeth 62 of the blade 38 are similar to and interlocked with the gear teeth 54 of the hub connector 48, as shown in FIG. 4. While the hub connector 48 is shown to include two gear teeth 54 and the blade 38 is shown include three gear teeth 62, the hub connector 48 and the blade 38 can include any number of interlocking gear teeth.

In operation, internal drive mechanisms (not shown) of the bone saw 12 are coupled to the hub 24 of the bone saw 12 to cause the hub 24 to oscillate back and forth about an axis (not shown) through the post 32 of the blade clamp 20. This output oscillating motion of the bone saw 12 is transferred from the oscillating hub 24 of the blade clamp 20 of the bone saw 12 to the hub connector 48 of the adapter 10. As such, the hub connector 48 oscillates or pivots through a particular angle predetermined by the internal drive mechanism of the bone saw 12. The output oscillating motion of the bone saw 12 defines back-and-forth pivoting movement through an angle of approximately 8 to 9°. The hub connector 48 is coupled to the oscillating hub 24 of the saw 12 and therefore oscillates back-and-forth with the hub 24 through an angle of approximately 8 to 9°. This oscillation of the hub connector 48 urges the blade 38 to oscillate about the pin 68 due to the geared relationship between the blade 38 and the hub connector 48. The geared relationship between the hub connector 48 and the blade 38 operates to reduce the angle through which the blade 38 oscillates through approximately 3.6°.

For example, a first distance 80 between a pivot point 82 of the hub adapter 48 and an interlocking position between the gear teeth 54, 62 is less than a second distance 82 between a pivot point 84 of the blade 38 and the interlocking position between the gear teeth 54, 62, as shown in FIG. 4. As shown, a gear ratio between the blade 38 and the hub connector 48 is 2:1, but any suitable gear ratio between the blade 38 and the hub connector 48 can be used in order to reduce the angle through which the blade 38 oscillates from the angle through which the hub 24 and hub connector 48 oscillate. It should be understood that the gear ratio may be altered by changing the first and/or second distances 80, 82 described above.

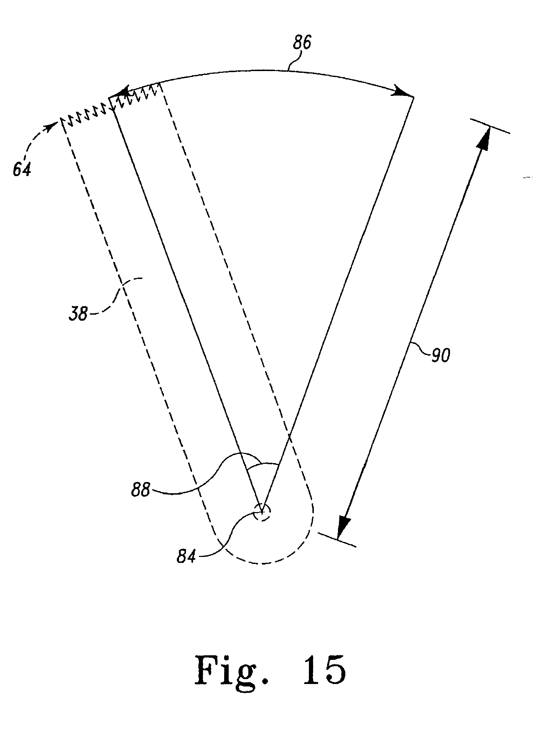

Preferably, the distal end 64 of the oscillating blade 38 travels back and forth through an arc length of 4.4 to 5.6 mm. The arc length is defined as the distance of travel of any point on the distal end 64 of the blade 38 as it travels through one sweep of motion of the blade 38 through a particular angle. The oscillating motion of the blade 38 is shown diagrammatically in FIG. 15. The arc length 86 of the oscillating motion of any point on the distal end 64 of the blade 38 is a function of an angle 88 (measured in radians) through which the blade 38 is moving and a length 90 of the blade 38 as measured from the pivot point 84 of the blade 38 to the distal end 64 of the blade 38. In other words, the length 90 of the blade 38 multiplied by the angle 88 through which the blade 38 is oscillating equals the arc length 86 of the oscillating motion of any particular point on the distal end 64 of the blade 38. Accordingly, altering the length 90 of the blade 38 and/or the angle 88 through which the blade 38 is oscillating will alter the arc length 86 of the oscillating motion of any point on the distal end 64 the blade 38.

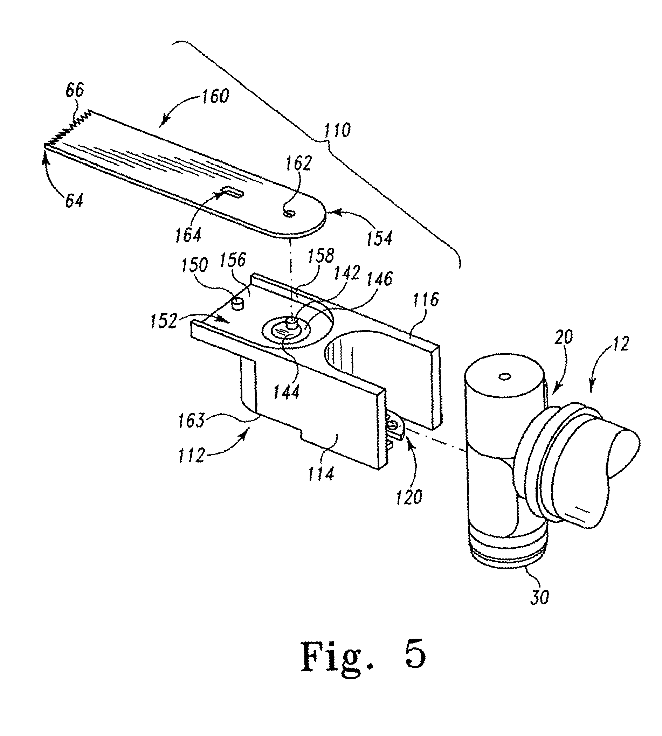

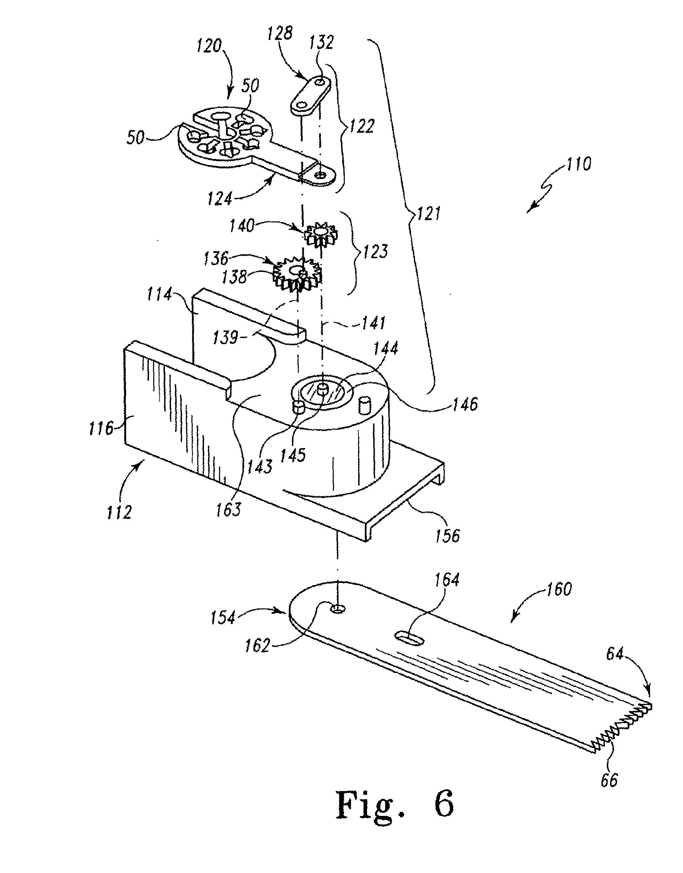

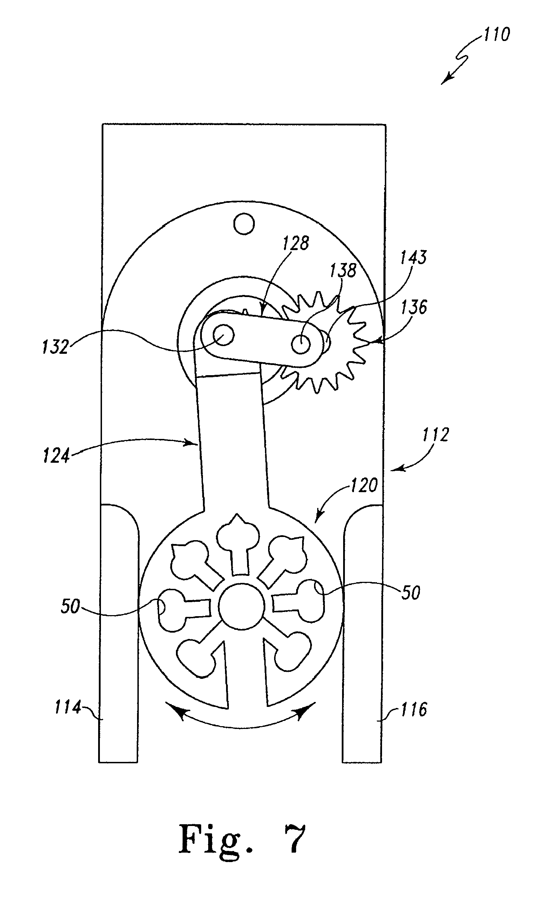

FIGS. 5 to 8 show another saw adapter 110 which can be coupled to the bone saw 12 in order to provide an orbital bone saw assembly. The adapter 110 operates to change the oscillating output motion of the bone saw 12 to orbital motion. The saw adapter 110 includes a housing 112 including two arms 114, 116 for coupling to the blade clamp 20 of the bone saw 12 similar to the manner in which the housing 40 of the saw adapter 10 (shown in FIGS. 2 to 4) is coupled to the bone saw 12. The saw adapter 110 further includes a driven mechanism 121 (see FIG. 6) coupled to the drive mechanism (not shown) of the bone saw 12 via the hub 24, of the bone saw 12. The driven mechanism 121 is coupled to the housing 112 and includes a hub connector 120 (shown in FIGS. 6 and 7) similar to the hub connector 48 described above in regards to the adapter 10. As such, the slots 50 of the hub connector 120 are able to receive the detents 26 of the hub 24.

The driven mechanism 121 of the saw adapter 110 further includes a linkage mechanism 122 coupled to the hub connector 120 and a gear assembly 123 coupled to the linkage mechanism 122. The linkage mechanism 122 includes a first link 124 coupled at a first end to the hub connector 120 for back and forth oscillating movement with the hub connector 120. Preferably, the first link 124 and the hub connector 120 are formed as a unitary structure, but the first link 124 and the hub connector 120 can be formed as separate structures coupled to each other. A second link 128 is pivotally coupled at a first end to the second end of the first link 124 by a pivot pin 132. The second end of the second link 128 is coupled to a first gear 136 of the gear mechanism 123 by a pin 138. The pin 138 is spaced-apart from a center pivot axis 139 of the gear 136 such that the movement of the second link 128 causes the gear 136 to pivot about the axis 139. The gear 136 pivots about a hub 143 coupled to the housing 112 and defining the pivot axis 139.

A second gear 140 of the gear mechanism 123 is interlocked with the first gear 136 and is urged by the first gear 136 to pivot about a center pivot axis 141. A hub 145 is coupled to the second gear 140 and pivots with the second gear 140 about the axis 141. A through-shaft 144 (see FIGS. 5 and 6) is coupled to the hub 145 to rotate with the second gear 140 and the hub 145 about the axis 141 as well. A bearing 146 surrounds the through-shaft 144 to reduce friction of the through-shaft 144 as it rotates about the axis 141. The through-shaft 144 extends through the housing 112 from a bottom surface 163 of the housing 112 to a recessed surface 156 (shown in FIG. 5) of the housing 112. As such, the hub 145 is coupled to a first end of the through-shaft 144. An output shaft or peg 142 (shown in FIG. 6) is coupled to a second or opposite end of the through-shaft 144 and is offset from a center of the through-shaft 144. As such, the output shaft 142 travels in a circular motion around the pivot axis 141. It is within the scope of this disclosure to space the output shaft 142 any suitable distance from the pivot axis 141 of the second gear 140 in order to adjust a diameter of the circular motion of the output shaft 142 as it travels around the axis 141.

The second gear 140 is smaller than the first gear 136. As such, the gear ratio between the first and second gears operates to increase the speed at which the second gear 140 rotates and at which the output shaft 142 rotates about the axis 141 of the second gear 140. Preferably, a gear ratio between the first gear 136 and the second gear 140 is approximately 1.8. In other words, the gear ratio of the gear mechanism 123 operates to increase the speed (RPM) of the bone saw assembly by a factor of about 1.8, but gear mechanisms defining different gear ratios can be used in order to generate a desired speed.

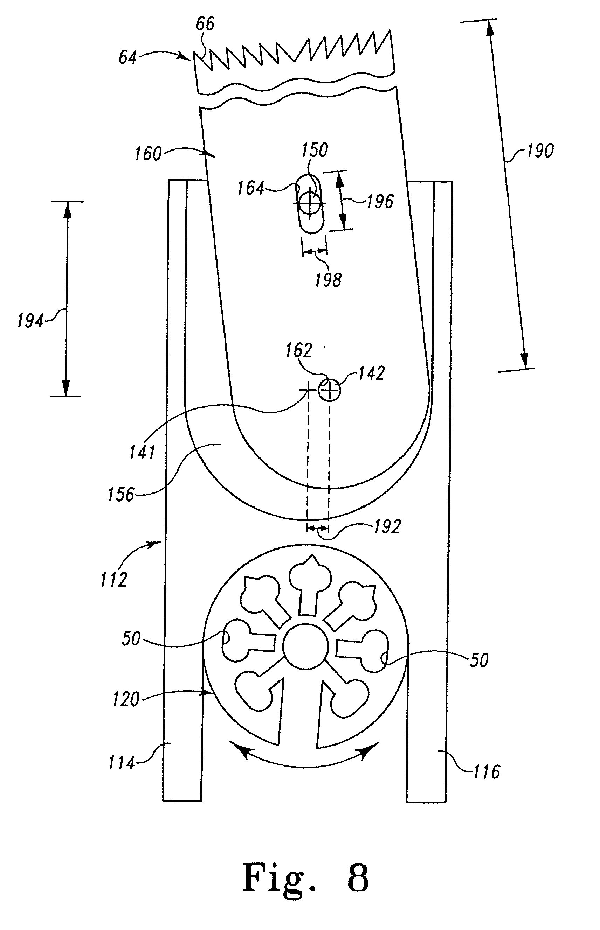

The saw adapter 110 further includes a guide pin 150, as shown in FIGS. 5 and 8, coupled to a recessed portion 152 of the housing 112. The recessed portion 152 of the housing 112 is sized to receive a proximal end 154 of a bone saw blade 160. The recessed portion 152 is defined by a recessed surface 156 of the housing 112 and a U-shaped side wall 158 of the housing 112 to contain the proximal end 154 of the blade 160 therein. Preferably, the saw blade 160 includes a first aperture 162 configured to receive the output shaft 142 of the driven mechanism 121 therein, as shown in FIG. 5. The blade 160 further includes a slot 164 spaced-apart from the aperture 162. The slot 164 extends longitudinally along a length of the blade 160. In other words, a longitudinal axis (not shown) of the slot 164 is parallel to a longitudinal axis (not shown) of the blade 160. The slot 164 is positioned to receive the guide pin 150 of the housing 112 in order to convert the circular motion of the blade 160 (provided by the output shaft 142) to an ellipse-like motion. As is discussed in greater detail below, the output motion of the distal end 64 of the blade 160 follows a path which is generally elliptical in shape.

In operation, therefore, the oscillating output motion from the drive mechanism of the oscillating bone saw 12 causes the hub connector 120 coupled to the blade clamp 20 of the saw 12 to oscillate with the hub 24 of the blade clamp 20 through the same angle that the hub 24 of the blade clamp 20 oscillates. As such, the first link 124, coupled to the hub connector 120, oscillates through this same angle as well. The oscillating motion of the first link 124 is translated to the second link 128 to cause the second end of the second link 128 to urge the first gear 136 to rotate about the hub 143 defining the pivot axis 139. As described above, rotation of the first gear 136 urges the second gear 140 to rotate about the hub 145 defining the pivot axis 141 thereby causing the output shaft 142 coupled to the second gear 140 at a position spaced-apart from the axis 141 to move in a circular motion about the axis 141. The blade 160 of the adapter 110 is coupled to the output shaft 142 such that the circular motion of the output shaft 142 urges the proximal end 154 of the blade 160 to move in the same circular motion. However, the guide pin 150 received through the slot 164 of the blade 160 causes the output motion of the distal end 64 of the blade 160 to follow an ellipse-like path.

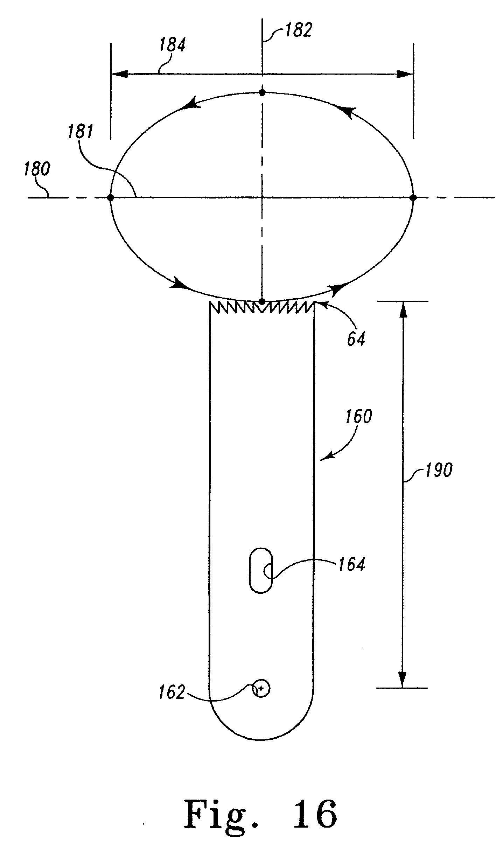

This orbital motion of the distal end 64 of the blade 160 may be elliptical, as shown diagrammatically in FIG. 16. However, it should be understood that the orbital motion of the blade 160 is not limited to an elliptical output motion, but may travel in any orbital motion or path of any shape which produces a symmetrical closed loop or curve. For example, oscillating motion of the distal end 64 of the blade 160 may not follow a "perfect" elliptical path, but rather may follow a symmetrical closed loop which forms an oblong, or ellipse-like shape.

As shown in FIG. 16, the orbital, or symmetrical closed curve, motion of any point on the distal end 64 of the blade 160 defines a major axis 180 and a minor axis 182. A major chord of a symmetrical closed curve is defined as the longest distance across the orbital path that is either perpendicular to the longitudinal axis of the blade or is parallel to the longitudinal axis of the blade. Accordingly, the major chord 181 of the orbital path shown in FIG. 16 lies along the major axis 180. However, the major axis may not always define the major chord. The major chord 181, therefore defines a length 184 which is preferably less than or equal to about 5.6 mm (0.220 inch). Preferably, the length 184 of the major chord 181 is between about 4.4 mm and about 5.6 mm (0.175 inch and 0.220 inch), or any other suitable length.

As shown in FIG. 16, the major axis 180 of the orbital motion or path is perpendicular to a longitudinal axis (not shown) of both the blade 160 and the slot 164 formed in the blade 160. However, the orbital path of the distal end 64 of the blade 160 can define a major axis that is parallel to the longitudinal axis of the blade. Further, the orbital path of the output motion of the blade 160 can define a major axis that is neither parallel or perpendicular to a longitudinal axis of the blade 160, but is rather angled relative to the longitudinal axis of the blade 160.

Many different dimensions affect the length 184 of the major chord of the orbital output motion of the blade 160. As shown in FIG. 8, for example, a length 190 from the centre of the aperture 162 to the distal end 64 of the blade 160, a distance or radius 192 between the centre of the output shaft 142 and the axis of rotation 141, and a distance 194 between the center of the guide pin 150 and the axis of rotation 141 may each be varied in order to affect the output motion of the blade 160 while still maintaining an output motion of the blade 160 which defines a major chord having a length 184 between about 4.4 and 5.6 mm.

The size and shape of the slot 164 and/or the guide pin 150 may affect the output motion of the distal end 64 of the blade 160. For example, the slot 164 defines a length 196 which is at least two times the distance 192 between the centre of the output shaft 142 and the axis of rotation 141 plus a diameter of the pin 150. Preferably, the slot 164 defines a width 198 at least equal to the diameter of the pin 150.

Preferably, the radial distance 192 between the output shaft 142 and the axis 141 is less than the distance 194 between the output shaft 142 and the guide pin 150. Further, this distance 194 is less than the length 190 of the blade 160 as measured between the aperture 162 of the blade 160 and the distal end 64 of the blade 160. As shown in FIG. 8, the axis of rotation 141 is aligned with the centre of the guide pin 150. Similar to the slot 164 of the adapter 110, the size and shape of the slot 268 and/or the guide pin 264 of the bone saw blade 261 of the adapter 210 may also affect the output motion of the distal end 64 of the blade 261. For example, the slot 268 defines a length 296 which is at least two times the distance 292 between the centre of the output shaft 242 and the axis of rotation 236 plus a diameter of the pin 264. The slot 268 defines a width 398 at least equal to the diameter of the pin 264.

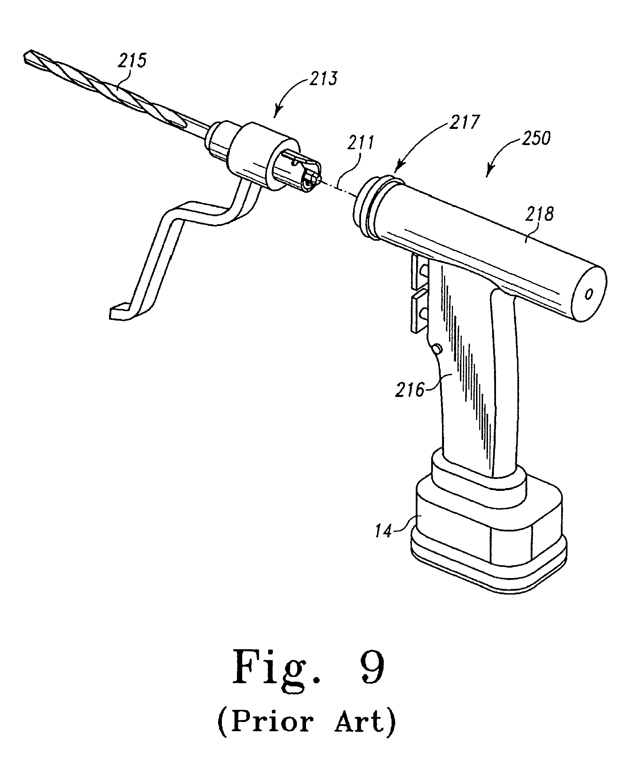

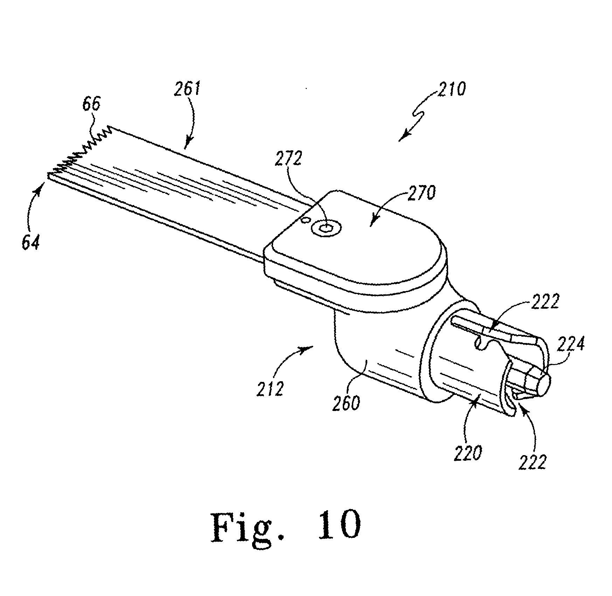

FIGS. 10 to 14 show a bone drill adapter 210 which can be coupled to a bone drill, such as the bone drill 250 shown in FIG. 9, in order to convert the rotational output motion of the bone drill 250 to an orbital motion, as discussed below. In effect, therefore, the adapter 210 and the bone drill 250 cooperate to provide an orbital bone saw assembly. The bone drill 250 may be any conventional bone drill such as for example the 7500 Drill/Reamer sold by MicroAire Surgical Instruments LLC of Charlottesville, VA. Similar to the oscillating bone saw 12 discussed above, the bone drill 250 includes an internal drive mechanism (not shown) which causes a chuck 217 or output drive shaft to rotate about an axis 211. Such chuck may be considered the tool-retention mechanism of the bone drill 250. The bone drill 250 includes the battery pack 14 coupled to a grip portion 216 and a head portion 218 coupled to the grip portion 216. Similar to the bone saw 12, the bone drill 250 may also be powered by a pneumatic source (not shown). Typically, a coupler 213 having a drill bit 215 is coupled to the head portion 216 to rotate the bit 215 about the axis 211.

FIGS. 10 to 12 show the adapter 210 with a housing 212 having a connector 220 configured to be received within the bone drill 250. The connector 220 includes first and second keyed slots 222 formed to receive a portion of the bone drill 250 therein in order to lock the adapter 210 to the bone drill 250. This coupling means of the connector 212 is generally the same as or similar to the connecting means of various drill coupler attachments, such as the attachment 213 shown in FIG. 9, used during orthopaedic surgery.

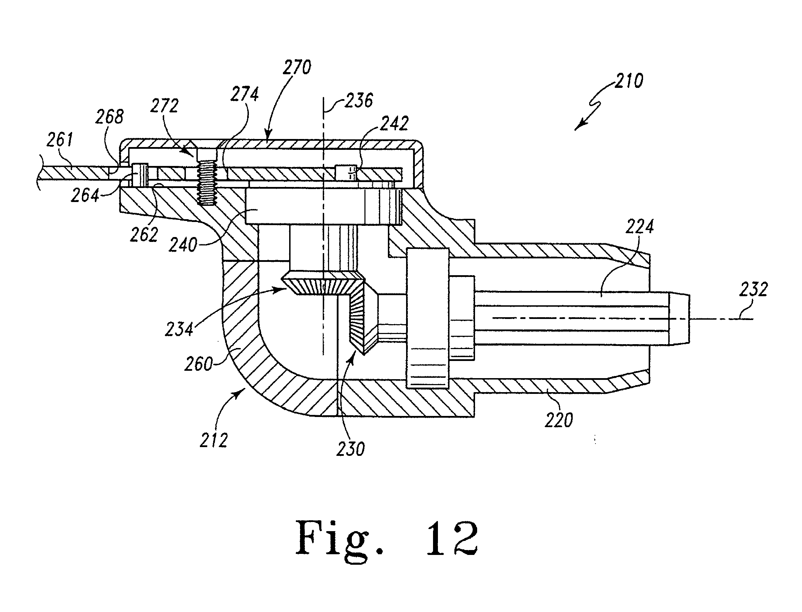

A drive shaft 224 of the adapter 210 is positioned within the connector 220 and is adapted to be received within a socket (not shown) of the drill 250 which produces the rotational output motion of the drill 250. As such, the drive shaft 224 of the adapter 210 is urged to rotate by the rotational motion of the socket of the drill 250. As further shown in FIG. 12, the drive shaft 224 of the adapter 210 is coupled to a first bevel gear 230 to urge the first bevel gear 230 to rotate with the drive shaft 224 about a first longitudinal axis 232. The first longitudinal axis 232 is collinear with the longitudinal axis 211 about which the socket of the drill 250 rotates. A second bevel gear 234 is coupled to the first bevel gear 230 and is positioned at a 90° angle to the first bevel gear 230 in order to change the direction of the rotational motion of the drive shaft 224 of the first bevel gear 230 by about 90°. Accordingly, the second bevel gear 234 rotates about a second, vertical axis 236 perpendicular to the first axis 232 about which the first bevel gear 230 rotates.

A disk 240 is coupled to the second bevel gear 234 in order to rotate with the second bevel gear 234 about the vertical axis 236. An offset output pin 242 or shaft is coupled to the disk 240. The output pin 242 is spaced-apart from the vertical axis of rotation 236, as shown in FIG. 12, such that the output pin 242 rotates around the axis 236 in a generally circular motion.

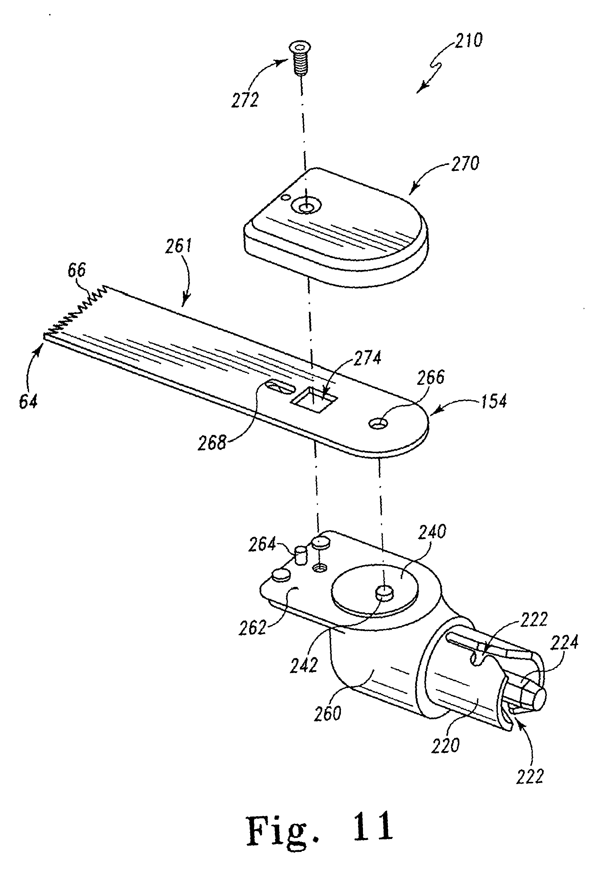

The housing 212 of the adapter 210 further includes a main body portion 260 coupled to the connector 220 and containing the bevel gears 230, 234 therein. The main body portion 260 defines an upper surface 262 formed to receive a blade 261 thereon (see FIGS. 11 and 12). A portion of the disk 240 and the output pin 242 extend through the upper surface 262 of the housing 212. Further, a guide pin 264 of the housing 212 is coupled to the upper surface 262 and is spaced-apart from the output pin 242. The blade 261 is similar to the blade 160 and includes an aperture 266 formed to receive the output pin 242 as well as a slot 268 having a longitudinal axis (not shown) which extends along a longitudinal axis of the blade 261 (not shown).

In operation, the aperture 266 of the blade 261 receives the output pin 242 to urge the proximal end 154 blade 261 to move in a circular motion with the pin or output shaft 242. However, the guide pin 262 of the housing 212 is received within the slot 268 of the blade 261 which causes the output motion of the distal end 64 of the blade 261 to follow an ellipse-like orbital motion defined by a symmetrical closed curve or loop. A cap 270 of the housing 212 is coupled to the main body portion 260 of the housing 212 by a threaded screw 272 received through another aperture 274 of the blade 260. The aperture 274 of the blade 261 is positioned between the slot 268 of the blade and the aperture 266 of the blade and is sized such that any motion of the blade 261 is not restricted.

Similar to the adapter 110 described above, the orbital motion of the blade 261 of the adapter 210 is not limited to an elliptical output motion, but may travel in any orbital motion or path which completes a symmetrical closed curve or loop. In other words, the orbital output motion of the blade 260 may be circular, elliptical, oval, or any other shape which produces a symmetrical closed loop or curve. Further, the length 184 of the major chord of the orbital motion of the blade 261 is preferably less than or equal to about 5.6 mm. In a preferred embodiment, the length 184 of the major chord is between about 4.4 mm and about 5.6 mm (0.175 to 0.200 inch), but the orbital motion of the blade 261 to define a major chord having any suitable size.

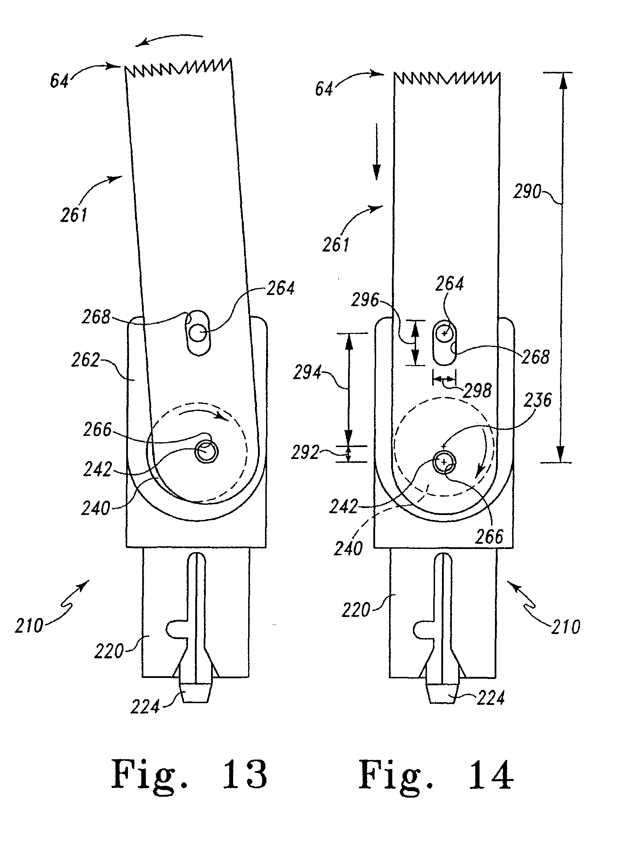

As with the adapter 110 described above, many different dimensions of the adapter 210 may affect the length 184 of the major chord of the orbital output motion of the distal end 64 of the blade 261. As shown in FIGS. 13 and 14, for example, a length 290 from the centre of the aperture 266 to the distal end 64 of the blade 261, a distance or radius 292 between the centre of the output shaft 242 and the axis of rotation 236, and a distance 294 between the centre of the guide pin 264 and the axis of rotation 236 may each be varied in order to affect the output motion of the blade 261.

The size and shape of the slot 268 and/or the guide pin 264 may also affect the output motion of the distal end 64 of the blade 261. Preferably, the radial distance 292 between the output shaft 241 and the axis 236 is less than the distance 294 between the output shaft 241 and the guide pin 264. Further, this distance 294 is less than the length 290 of the blade 261 as measured between the aperture 266 of the blade 261 and the distal end 64 of the blade 261. As shown in FIGS. 13 and 14, the axis of rotation 236 is aligned with the centre of the guide pin 264.

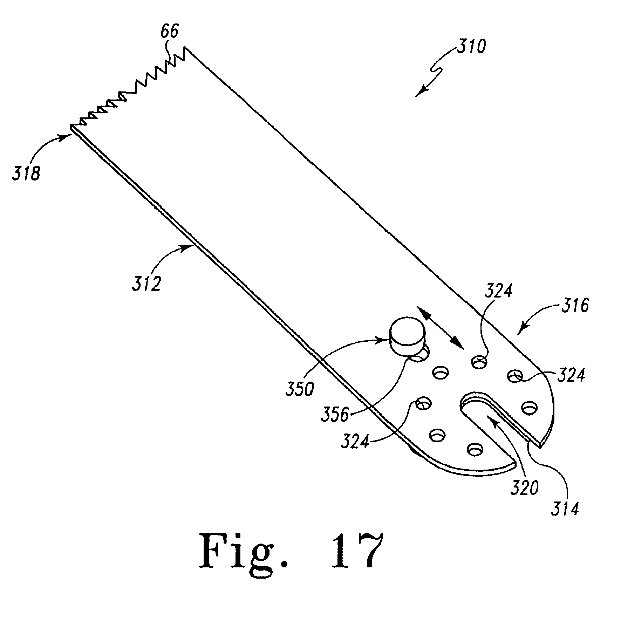

FIGS. 17 to 20 show an adapter 310 which includes a bone saw blade 312 and a dual-mode adjuster plate 314 coupled to the bone saw blade 312. The adapter 310 is configured to be coupled to an oscillating bone saw (not shown) in order to modify the oscillating output motion of the saw. When coupled to an oscillating bone saw, the adapter 310 provides a dual-mode oscillating bone saw assembly which operates to provide two different oscillating output motions of the distal end of the blade 312 which each define a different arc length. The adapter 310 (including the bone saw blade 312 and the adjuster plate 314) can be coupled to the Sagittal Saw provided by Stryker Corporation (Kalamazoo, MI), or to other similar oscillating saws.

The bone saw blade 312 includes a proximal end 316 configured to be coupled to an oscillating bone saw (not shown) and a distal end 318 having saw teeth 66. As shown, the proximal end 316 of the bone saw blade 312 includes a U-shaped slot 320 defining a pivot point 322 of the bone saw blade 312 and seven apertures or openings 324 positioned around the slot 320. The slot 320 of the bone saw blade 312 is configured to receive a vertical post (not shown), similar to the central post 32 of the oscillating bone saw 12 shown in FIG. 1, while the apertures 324 of the bone saw blade 312 are configured to receive an array of detents (not shown) similar to the array of detents 26 protruding from the top surface 28 of the hub 24 of the bone saw 12 in much the same way that the blade 22 shown in FIG. 1 is coupled to the bone saw 12. As is discussed in greater detail below, the diameter 330 of each aperture 324 is approximately 2.7 mm (0.108 inch) while the diameter (not shown) of the respective detent (not shown) received within each aperture 324 is smaller than 2.7 mm (0.108 inch). In other words, the diameter of the detent of the oscillating bone saw to which the blade 312 is coupled is generally smaller than the diameter 330 of each respective aperture 324 of the blade 312 through which the detent is received. As such, each aperture 324 is sized such that a minimum clearance exists between an outer surface of the detent and a corresponding surface of each respective aperture 324. For example, the diameter of each detent may be 2.5 mm (0.1 inch) in order to provide a clearance of 0.2 mm (0.008 inch) between the detent and the surface of each corresponding aperture 324.

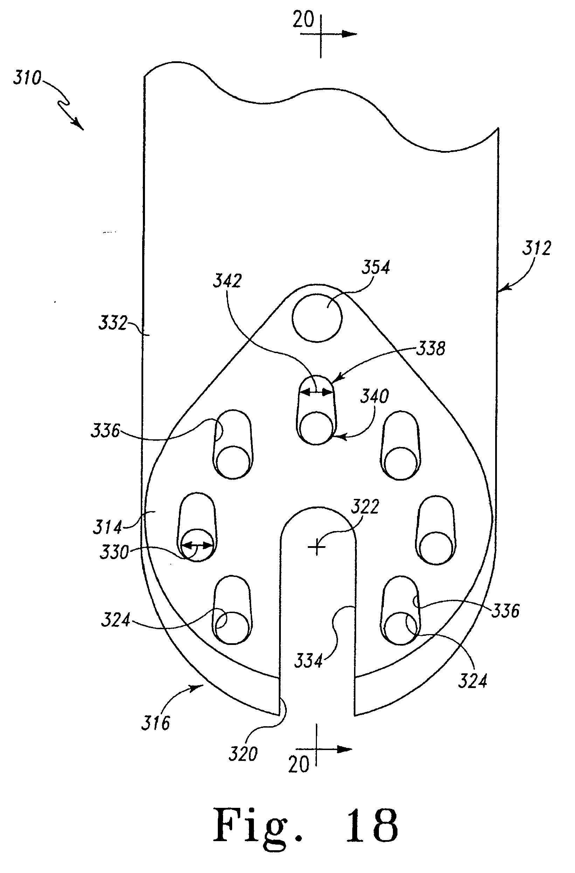

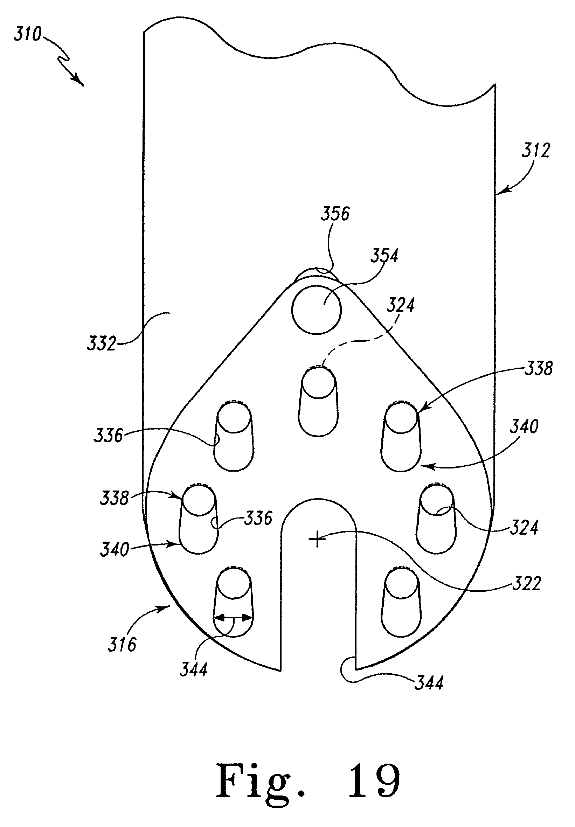

FIGS. 18 to 20 show the adjuster plate 314 of the adapter 310 located adjacent a bottom surface 332 of the bone saw blade 312 and includes a U-shaped slot 334 similar in size and aligned with the U-shaped slot 320 of the bone saw blade 312. Further, the adjuster plate 314 includes seven slots or openings 336 each defining a longitudinal axis (not shown) parallel to the longitudinal axis (not shown) of the blade 312. Each slot 336 is generally tear-drop shaped such that a distal end 338 of each slot 336 is narrower than a proximal end 340 of each slot 336. The width 342 (shown in FIG. 18) of the distal end 338 of each slot 336 is approximately 2.5 mm (0.1 inch) while the width 344 (shown in FIG. 19) of the proximal end 340 of each slot 336 is approximately 2.8 mm (0.112 inch).

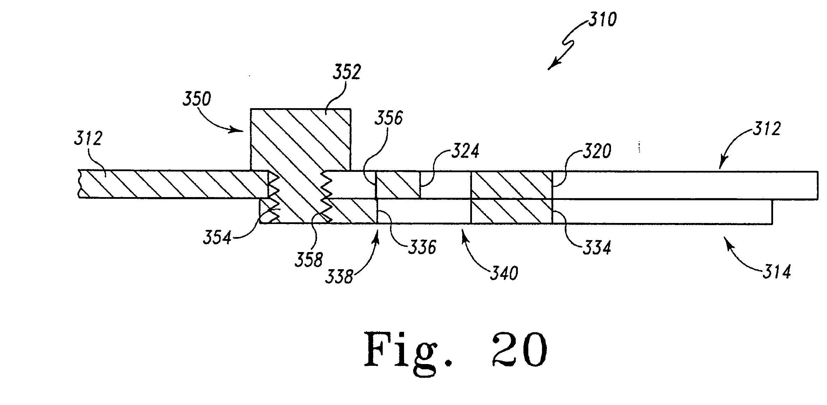

The adapter 310 further includes a knob 350 coupled to both the blade 312 and the adjuster plate 314, as shown in FIG. 20, for example. The knob 350 includes a head 352 and a threaded post 354 received through both a slot 356 formed in the bone saw blade 312 and a threaded aperture 358 formed in the adjuster plate 314. The slot 356 formed in the blade 312 defines a longitudinal axis parallel to and aligned with the longitudinal axis of the blade 312. The knob 350 is coupled to the adjuster plate 314 and movable relative to the blade 312 within the slot 356 formed in the blade 312, as is discussed in greater detail below. The knob 350 may also be tightened and loosened against the blade 312 simply by rotating the knob 350 clockwise or counterclockwise in order to allow the surgeon or other technician to secure the position of the adjuster plate 314 relative to the blade 312.

The adjuster plate 314 is movable between a first position shown in FIGS. 17 and 18 and a second position shown in FIG. 19. In the first position, the apertures 324 of the bone saw blade 312 are generally aligned with the proximal end 340 of the slots 336 of the adjuster plate 314. As noted above, the width 344 of the proximal end 340 of each slot 336 of the adjuster plate 314 is approximately 2.7 mm (0.108 inch) while the diameter 330 of each aperture 324 of the blade 312 is approximately 2.8 mm (0.112 inch). As such, the adjuster plate 314 is not positioned to obscure any portion of the apertures 326 of the blade 312 when the adjuster plate 314 is in the first position.

During operation of the oscillating bone saw (not shown) with the adapter 310 coupled thereto and the adjuster plate 314 in the first position, the detents of the oscillating bone saw are urged by the internal drive mechanism (not shown) of the oscillating saw to oscillate back and forth about a pivot point substantially aligned with the pivot point 322 of the U-shaped slot 320. Such operation is similar to that described above with respect to the oscillating bone saw 12 shown in FIG. 1. The adapter 310, which is received on the detents, is therefore urged to oscillate back and forth about the pivot point 322 as well. However, the larger diameter 330 of each aperture 326 of the bone saw blade 312 relative to the detents received therein provides for extra radial travel of the each detent relative to the blade 312. In other words, the larger diameter 330 of each aperture 326 provides for lost motion of the respective detent within the aperture 324. For example, each detent of the bone saw is moved at least 0.2 mm (0.008 inch) about the pivot point relative to the bone saw blade. As such, the blade 312 is not urged to oscillate through the entire range of motion through which the detents oscillate. For example, when the adjuster plate 314 is in the first position, the detents may oscillate through an arc of approximately 8° whereas the adjuster 310 may be urged to oscillate through an arc of approximately 3.6° due to the larger diameter apertures 324 of the bone saw blade 312.

As noted above, the adjuster plate 314 is also movable to a second position shown in FIG. 19. In the second position, the adjuster plate 314 is positioned further proximally relative to the bone saw blade 312 than when in the first position. Further, the apertures 324 of the bone saw blade 312 are generally aligned with the distal end 338 of the respective slots 336 of the adjuster plate 314. As noted above, the width 342 of the distal end 338 of each respective slot 336 is approximately 2.54 mm (0.100 inch) which is smaller than the 2.74 mm (0.108 inch) width of the proximal end 340 of each respective slot 324. As such, when the adjuster plate 314 is in the second position, a portion of the adjuster plate 314 obscures the apertures 324 of the bone saw blade 312 in order to effectively reduce the size of the aperture through which the detents of the oscillating bone saw are received.

Accordingly, the usable size of the diameter of each aperture 324 of the bone saw blade 312 becomes generally equivalent to the size of the diameter of each detent to be received within the respective aperture. As such, during operation of the bone saw, little or no oscillating motion of the detents about the pivot point 322 is lost. In other words, as the detents oscillate back and forth, the adapter 310 is urged to oscillate back and forth through generally the same range of motion. As such, if the detents of the bone saw oscillate through an arc of approximately 8°, the blade 312 will similarly be urged to oscillate through an arc of approximately 8°.

As noted above, therefore, the adjuster plate 314 is movable relative to the bone saw blade 312 between first and second positions in order to provide an adapter having two modes of operation. In first mode of operation, when the adjuster plate 314 is in the first position, the oscillating output motion of the bone saw is adapted such that the bone saw blade 312 is urged to oscillate through an arc of approximately 3.6°. By reducing the angle through which the blade 312 oscillates, the arc length of the oscillating motion of the distal end 318 of the blade 312 is reduced as well. Preferably, the oscillating motion of the distal end of the blade 312 of the adapter 310 travels through an arc length of 4.4 to 5.6 mm when the adjuster plate 314 is in the first position.

In the second mode of operation, when the adjuster plate 314 is in the second position, the bone saw blade 312 is urged to oscillate through approximately the same angle as the detents of the oscillating bone saw to which the adapter 310 is coupled. In other words, in the second mode of operation, the bone saw blade 312 is urged to oscillate through an angle of approximately 8°. As such, in the second mode of operation, the surgeon may use the bone saw without adapting the oscillating output motion of the bone saw.

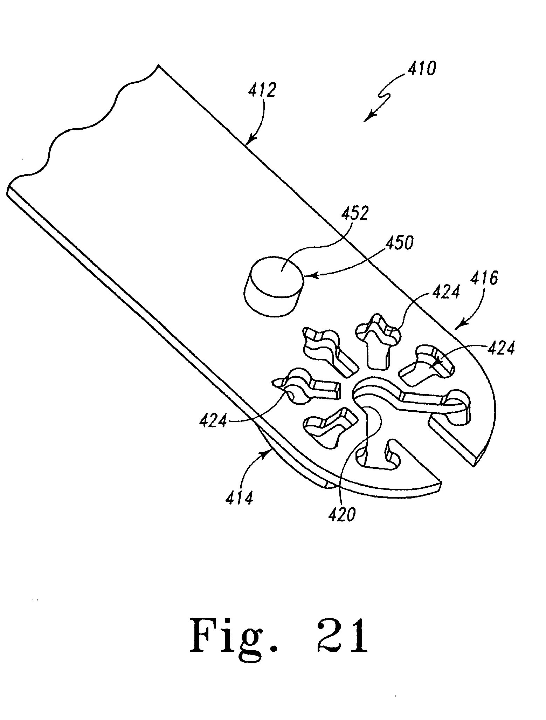

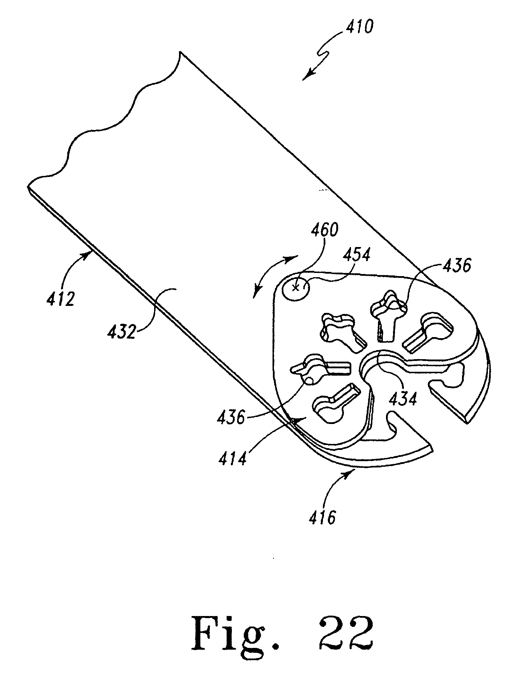

FIGS. 21 and 22 show another adapter 410 is provided for use with an oscillating saw such as that sold under the trade mark Hall PowerPro by ConMed Linvatec. Similar to the adapter 310, the adapter 410 includes a bone saw blade 412 and a dual-mode adjuster plate 414 coupled to the bone saw blade 412.

The bone saw blade 412 includes a proximal end 416 configured to be coupled to an oscillating bone saw (not shown) and a distal end (not shown) having saw teeth (not shown). The proximal end 416 of the bone saw blade 412 includes an array of slots or openings 424 formed to receive an array of detents of the oscillating bone saw (not shown) in order to couple the bone saw blade 412 to the bone saw. The array of slots 424 is similar to the array of slots 34 of the bone saw blade 22 shown in FIG. 1 and array of slots 50 of the hub connector 48 of the adapter 10 shown in FIGS. 3 and 4. Such arrays of slots 34, 50 are formed to receive the array of detents 26 of the blade clamp 20 of the bone saw 12 shown in FIGS. 1 and 2. As such, the proximal end 416 of the blade 412 is able to receive the array of detents of the oscillating saw to which the adapter 410 is configured to be coupled. Specifically, a centre U-shaped cut-out 420 of the bone saw blade 412 is configured to receive a vertical post (not shown) of the saw about which the blade 412 oscillates.

FIG. 22 shows the adjuster plate 414 of the adapter 410 located adjacent a bottom surface 432 of the bone saw blade 412 and includes a curved cut-out portion 434 similar in size and aligned with the U-shaped cut-out 420 of the bone saw blade 412. Further, the adjuster plate 414 includes an array of five slots or openings 436 each similar in shape to a corresponding one of the array of slots 424 of the bone saw blade 412. The size of each slot 436 within the array of slots of the adjuster plate 414 is different from the size of each corresponding slot 424 within the array of slots of the bone saw blade 412. In particular, the area of each slot 424 of the blade 412 is greater than the area of each slot 436 of the adapter plate 414.

The adapter 410 includes a knob 450 coupled to both the blade 412 and the adjuster 414, as shown in FIG. 21, for example. The knob 450 includes a head 452 and a threaded post 454 (shown in FIG. 22) received through an aperture (not shown) formed in the bone saw blade 412 and a threaded aperture (not shown) formed through the adjuster plate 414. The knob 450 is coupled to the adjuster plate 414 and rotatable relative to the bone saw blade 412 such that rotating the knob in a clockwise or counterclockwise direction operates to move the adjuster plate 414 relative to the blade 412. Specifically, the knob 450 operates to rotate the adjuster plate 414 about pivot point 460 (shown in FIG. 22) relative to the bone saw blade 412 in order to adjust the effective size of the slots 424 of the blade 412, as discussed below. The knob 450 may also be tightened and loosened against the blade 412 in order to allow the surgeon or other technician to lock or secure the adjuster plate 414 in a particular location relative to the blade 412.

Similar to the adjuster plate 314, the adjuster plate 414 is movable between a first position and a second position. In the first position, the slots 424 of the bone saw blade 412 are generally aligned with the slots 436 of the adjuster plate 414 such that the adjuster plate 414 does not operate to obscure any portion of the slots 424 of the blade 412. Preferably, the size of each detent of the oscillating bone saw is smaller than the size or area of each respective slot 424 of the blade 412 through which such detent is received.

During operation of the oscillating bone saw with the adapter 410 coupled thereto and the adjuster plate 414 in the first position, the detents of the oscillating bone saw are urged by the internal drive mechanism (not shown) of the oscillating saw to oscillate back and forth about a pivot point substantially aligned with the centre slot 420. Such operation is similar to that described above with respect to the oscillating bone saw 12 shown in FIG. 1. The adapter 410, which is received on the detents, is therefore urged to oscillate back and forth about such pivot point as well. However, the larger size or area of each slot 424 of the bone saw blade 412 relative to the detents received therein provides for extra radial travel of each detent relative to the blade 412. In other words, the larger sized slots 424 provide for lost motion of the respective detent within each slot 424. As such, the blade 412 is not urged to oscillate through the entire range of motion through which the detents oscillate. For example, when the adjuster plate 414 is in the first position, the detents may oscillate through an angle of approximately 8° whereas the adjuster 410 may be urged to oscillate through an angle of approximately 3.6° or less due to the larger sized slots 424 of the bone saw blade 412.

As noted above, the adjuster plate 414 is also movable to a second position. In the second position, the adjuster plate 414 is rotated about the pivot point 460 in order to position such that a portion of the adjuster plate 414 obscures a portion of the slots 424 of the bone saw blade 412. In this second position, therefore, the size or area of the slots through which the detents of the oscillating bone saw are received is reduced.

When the adjuster plate 414 is in the second position, the effective size of each partially-occluded slot 424 is reduced. As such, the effective or usable size or area each slot 424 of the bone saw blade 412 becomes generally equivalent to the size of the each detent to be received within the respective aperture. As such, during operation of the bone saw, little or no oscillating motion of the detents about the pivot point 420 is lost. In other words, as the detents oscillate back and forth, the adapter 410 is urged to oscillate back and forth through generally the same range of motion. As such, when the adjuster plate is in the second position, if detents of the bone saw are urged to oscillate through an angle of approximately 8°, for example, the blade 412 of the adjuster 310 is urged to oscillate through an angle of approximately 8° as well.

As noted above, therefore, the adjuster 414 is movable relative to the bone saw blade 412 between first and second positions in order to provide an adapter having two modes of operation. In the first mode of operation, when the adjuster 414 is in the first position, the oscillating output motion of the bone saw is adapted such that the bone saw blade 412 is urged to oscillate through an angle of approximately 3.6° or less. By reducing the angle through which the blade 412 oscillates, the arc length of the oscillating motion of the distal end of the blade 412 is reduced as well. Preferably, the oscillating motion of the distal end of the blade 412 of the adapter 410 travels through an arc length of about 4.4 to about 5.6 mm (0.175 to 0.220 inch) when the adjuster plate 414 is in the first position.

In the second mode of operation, when the adjuster plate 414 is in the second position, the bone saw blade 412 is urged to oscillate through approximately the same arc angle as the detents of the oscillating bone saw to which the adapter 410 is coupled. In other words, in the second mode of operation, the bone saw blade 412 is urged to oscillate through an arc angle of approximately 8°. As such, in the second mode of operation, the surgeon may use the bone saw without adapting the oscillating output motion of the bone saw.

An adapter can provide a first mode of operation whereby the output arc angle of the oscillating saw to which the adapter is coupled is modified or reduced such that the arc angle of the oscillating motion of the bone saw blade is less than that which is output by the oscillating saw. An adapter to provide a second mode of operation whereby the output arc angle of the oscillating saw to which the adapter is coupled remains generally unchanged such that the arc angle of the oscillating motion of the bone saw blade is generally equal to that which is output by the oscillating saw. Such an adapter provides the surgeon with a choice between two modes of operation.

It may be desirable, for example, for the surgeon or other technician to be able to use an oscillating bone saw assembly which is capable of producing two different output motions of the bone saw blade 412. For example, a surgeon may choose to position the adjuster in the first mode of operation when many soft tissues are adjacent to or surrounding the bone being cut. Alternatively, the surgeon may wish to switch to a bone saw assembly which provides a greater arc length during certain other portions of the surgical procedure.

The invention can be used in retrofit assemblies which include an adapter and an existing oscillating saw or bone drill. The invention also provides a bone saw assembly (either oscillating or orbital) which is built or manufactured to be used with a blade (rather than the adapters disclosed herein) but which operates similarly to the retrofit assemblies discussed above. In other words, instead of a retrofit adapter, the concepts discussed above in regards to assisting surgeons in reducing damage to soft tissue may be applied to an original manufactured bone saw assembly as well.

As discussed above, decreasing the arc length or major chord of the oscillating or orbital output motion of the bone saw blade can help to reduce the potential for damage to soft tissues surrounding the particular bone being cut. However, decreasing the arc length or major chord of the blade motion can also increase the time required to make a particular bone cut. Further, the speed and torque of the bone saw or bone saw assembly may further influence the cutting efficiency of the bone saw blade. For example, many current bone saw assemblies operate at a speed of approximately 11,000 cycles per minute. For the bone saw assemblies disclosed herein which produce an arc length or major chord of 4.4 to 5.6 mm (0.175 to 0.220 inch), it should be noted that the speed may be increased to approximately 20,000 to 25,000 cycles per minute in order to provide the bone cutting efficiencies of many typical bone saw assemblies whose bone saw blades produce an output motion having a larger arc length and/or major chord, for example. Further, the bone saw assemblies of the invention operate to provide an arc length or major chord less than 4.4 mm (0.175 inch) and which operate at a speed greater than 11,000 cycles per minute in order to compensate for any loss of efficiency which may be created due to the reduced arc length or major chord of the blade motion. In other words, the arc length or major chord of the output motion of the blade may further be reduced while increasing the speed of the bone saw assembly.

The invention can be applied to bone saw blades which are made from a variety of suitable materials, including for example stainless steel, aluminum or titanium (for light weight), or other suitable metals. A light weight blade has the advantage of reducing the mass moment of inertia of the bone saw blade during use, so that the vibrations of any bone saw used with such a bone saw blade may be reduced as well as the level of noise produced by the bone saw assemblies. Further, the oscillating output motion of a lighter blade may produce a smaller arc angle and undergo less deflection than that of an otherwise equivalent heavier blade. Deflection, or the out-of-plane bending of the blade during operation of the bone saw, may also be reduced by reducing the length of the blade. For example, it has been found that such blade deflection may be reduced up to 40% by reducing the length of a standard PFC Sigma blade made by DePuy Products, Inc. (Warsaw, IN) from 106 mm (4.165 inch) to approximately 89 mm (3.5 inch).

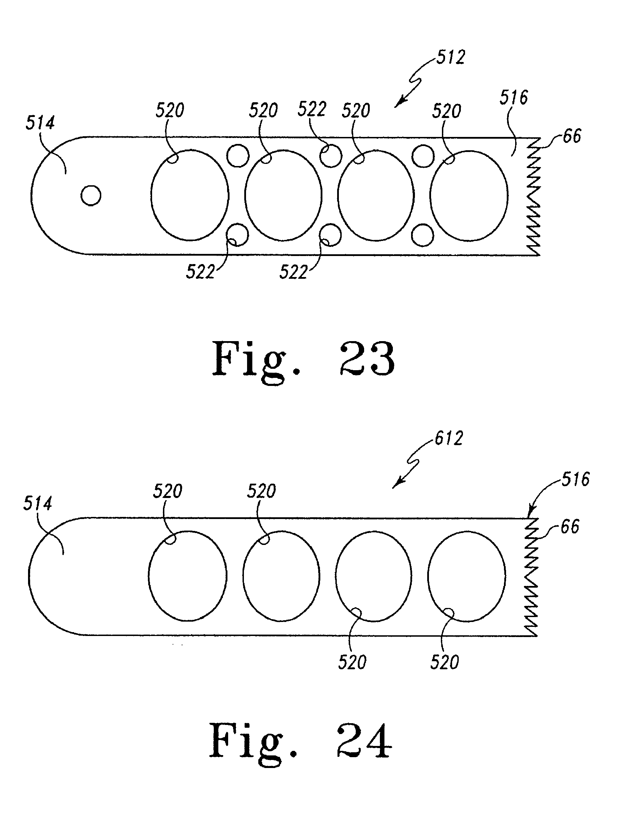

As noted above, manufacturing bone saw blades from aluminum or titanium reduces the mass of the bone saw blade which, in turn, reduces the mass moment of inertia of the bone saw blade in order to reduce vibration of the bone saw to which the blade is attached. The mass of the bone saw blade may also be reduced by removing material from the body of the blade, or put another way, by forming holes along the length of the blade. Looking to FIG. 23, for example, a bone saw blade 512 is shown. The bone saw blade 512 may be adapted for use with any of the above-referenced bone saws or bone saw adapters. Preferably, the bone saw blade is made from a titanium alloy such as Ti-6-Al-4V, for example, and includes a hub or proximal end 514 to be coupled to a bone saw or bone saw adapter and a distal end 516 having saw teeth 66.

Various cut-out portions or holes are provided in the bone saw blade 512 along the length of the bone saw blade 512 between the proximal end 514 and the distal end 516. For example, four large cut-out portions 520 are linearly spaced along the length of the bone saw blade 512. Each large cut-out portion 520 is generally oval in shape, but may be circular, square, rectangular, or any other suitable shape, for example. Further, while four large cut-out portions 520 are provided in the bone saw blade 512, any number of large cut-out portions 520 can be provided in the bone saw blade 512.

The bone saw blade 512 further includes six small cut-out portions 522. For example, a first array including three of the six small cut-out portions 522 can be linearly spaced along the length of the bone saw blade 512 such that each small cut-out portion 522 of the first array is positioned between the large cut-out portions 520. Similarly, a second array including three of the six small cut-out portions 522 can be linearly spaced along the length of the bone saw blade 512 such that each small cut-out portion 522 of the second array is positioned between the large cut-out portions 520 and is laterally aligned with a small-cut out portion 522 of the first array. Preferably, the first array of small cut-out portions 522 is positioned off-centre or medially from the longitudinal axis (not shown) of the blade 512 while the second array of small cut-out portions 522 is positioned laterally from the longitudinal axis. Preferably, the small cut-out portions 522 are circular in shape, but may be oval, square, rectangular, or any other suitable shape.

These large and small cut-out portions 520, 522 operate to reduce the mass of the blade 512 in order to reduce the mass moment of inertia of the blade 512. As noted above, such a reduction in the mass moment of inertia of the blade 512 may operate to reduce the overall vibration of the bone saw assembly. A reduction in vibrations may also operate to increase the stability of the bone saw assembly during use. Further, the large and small cut-out portions 520, 522 may be shaped and positioned to enhance the strength and stiffness of the blade 512 and to decrease the drag effect of the blade 512 on the bone as the blade 512 cuts through the patient's bone. Drag may be reduced by cutting down on the amount of surface area in contact with the bone as the bone is cut by the blade. Such a reduction in drag may also operate to decrease the amount of friction, energy lost, and heat generated.

FIG. 24 shows another bone saw blade 612 which can be used with any of the bone saws or bone saw adapters disclosed herein. The bone saw blade 612 is similar to the bone saw blade 512. As such, like reference numerals are used to denote like components. Preferably, the bone saw blade 612 includes the four large cut-out portions 520 and does not include the six small cut-out portions 522 of the bone saw blade 512. Preferably, the bone saw blade 612 is made from aluminum, but other materials might be suitable. Because aluminum is a softer metal than the titanium alloy from which the bone saw blade 512 is made, the six small cut-out portions have been removed in order to increase the stiffness or rigidity of the blade 612. Due to the disparity in the densities of aluminum and such a titanium alloy, the mass of the bone saw blade 612 made from aluminum is lighter than the bone saw blade 512 made from the titanium alloy.

In general, bone saw blades, such as the bone saw blades disclosed herein, undergo large amounts of stress during bone cutting. As such, in order to reinforce the bone saw blades 512, 612 discussed above which are made from less dense materials such as aluminum and a titanium alloy, a case-hardening process may be used in order to increase the hardness of the bone saw blade without substantially increasing the mass of the blade itself. Whyco Finishing Technologies LLC (Thomason, CT) provide such a suitable case-hardening process which is marketed under the trade mark CeraFuse. This coating process uses a micro-arc oxidation technology which generally removes the outer layer of the material to be coated (i.e., the bone saw blade) by polishing, tumbling, or another method. Because this process changes the property of the outer layer of the original substrate, instead of simply adding a coating on top of the original substrate, the coating process is able to suitably harden the titanium alloy and/or aluminum bone saw blades 512, 612 for use with bone saws. For example, after such ceramic processing, the aluminum and titanium alloy blades 512, 612 may be as hard as or harder than traditional stainless steel bone saw blades. Preferred coating processes should preferably provide wear resistance, protection from thermal and corrosive damages, and additional resistance to electricity to the substrate. Such a resistance to heat may operate to reduce any necrosis of the bone caused by heat which may be generated during bone-cutting.

It should be appreciated that certain of the adapter designs disclosed herein (e.g., the adapters 310, 410 which utilize lost motion) may produce different output motions when engaged with bone relative to when operating freely. For example, the arc length of the distal end of a given adapter may be between 4.4 and 5.6 mm when engaged with bone, but the arc length of the same adapter may be outside of this range when operating free of the bone.

## Claims

1. An oscillating bone saw assembly comprising:
an oscillating bone saw including a drive mechanism, and
a bone saw blade coupled to the drive mechanism, in which (i) the arc length of the oscillating motion of a distal end of the blade is about 4.4 to about 5.6 mm (0.175 to 0.220 inch), and (ii) the oscillating motion of the bone saw blade defines an angle of approximately 3.6°.

2. The oscillating bone saw assembly of claim 1, in which a length of the bone saw blade between a pivot point of the bone saw blade and the distal end of the bone saw blade is approximately 89 mm (3.5 inch).

3. An orbital bone saw assembly for producing an orbital output motion comprising:
a drive mechanism, and
a bone saw blade coupled to the drive mechanism, in which the orbital output motion of the bone saw blade defines a major chord having a length of about 4.4 to about 5.6 mm.

4. The orbital bone saw assembly of claim 3, in which the length of the major chord of the orbital output motion of the bone saw blade is about 4.4 to about 5.6 mm.

5. The orbital bone saw assembly of claim 3, in which a longitudinal axis of the bone saw blade is perpendicular to the major chord defined by the orbital output motion of the bone saw blade.

6. The orbital bone saw assembly of claim 3, further comprising an oscillating bone saw in which the oscillating bone saw includes the drive mechanism.

7. The orbital bone saw assembly of claim 3, further comprising a bone drill in which the bone drill includes the drive mechanism.

8. An adapter configured to be coupled to a blade clamp of an oscillating bone saw for modifying oscillating output motion of the oscillating bone saw, the adapter comprising:
a housing configured to be coupled the blade clamp of the oscillating bone saw,
a hub connector configured to be coupled to a hub of the blade clamp of the oscillating bone saw, and
a bone saw blade pivotally coupled to the housing to define a pivot point, the bone saw blade having a proximal end coupled to the hub connector such that the angle of the oscillating motion of a distal end of the bone saw blade is less than the angle of the oscillating output motion of the oscillating bone saw.

9. An adapter configured to be coupled to a blade clamp of an oscillating bone saw for converting oscillating output motion of the oscillating bone saw to orbital motion, the adapter comprising:
a housing configured to be coupled to the blade clamp of the oscillating bone saw,
a driven mechanism coupled to the housing and configured to be coupled to a drive mechanism of the blade clamp of the oscillating bone saw to convert oscillating output motion of the oscillating bone saw to orbital motion, and
a bone saw blade coupled to the driven mechanism.

10. An adapter configured to be coupled to a chuck of a bone drill for converting rotational output motion of the bone drill to orbital motion, the adapter comprising:
a first bevelled gear configured to be coupled to an output drive shaft of the chuck of the bone drill to define a first axis of rotation,
a second bevelled gear coupled to the first bevelled gear and positioned to define a second axis of rotation perpendicular to the first axis of rotation, and
a bone saw blade having a proximal end coupled to the second bevelled gear to define a pivot point of bone saw blade.

11. An adapter configured to be coupled to a blade clamp of an oscillating bone saw for modifying oscillating output motion of the oscillating bone saw, the adapter comprising a bone saw blade including an opening configured to receive a detent of the blade clamp, the opening being sized such that a clearance of at least 0.2 mm (0.008 inch) exists between an outer surface of the detent and a corresponding surface of the opening.
